# EUROPEAN PATENT APPLICATION

(11) **EP 4 588 486 A1**
(43) Date of publication of application: **23.07.2025**
(21) Application number: 23865432.1
(22) Date of filing: 08.09.2023
(51) Int. Cl.: A61K 45/00, A61P 43/00, A61P 25/28, A61K 31/351

(54) **PHARMACEUTICAL COMPOSITION FOR TREATING BRAIN DISEASE, NEURONAL MIGRATION PROMOTER, AND USE THEREOF**

(30) Priority: 12.09.2022 JP 2022144880
(71) Applicant: PUBLIC UNIVERSITY CORPORATION NAGOYA CITY UNIVERSITY, Nagoya-shi, Aichi 467-8601 (JP)
(72) Inventor: SAWAMOTO Kazunobu, Nagoya-shi, Aichi 467-8601 (JP); MATSUMOTO Mami, Nagoya-shi, Aichi 467-8601 (JP)
(74) Representative: Kuhnen & Wacker Patent- und Rechtsanwaltsbüro PartG mbB
(86) International application number: PCT/JP2023/032912
(87) International publication number: WO 2024/058077

(57) **Abstract**

Provided is a technology capable of promoting migration of neurons. This pharmaceutical composition for treating a brain disease includes a neuraminidase inhibitor, this neuronal migration promoter includes a neuraminidase inhibitor, and this method of promoting migration of neurons includes a step of bringing a neuraminidase inhibitor into contact with neurons.

## Description

### [Technical Field]

The present disclosure relates to a pharmaceutical composition for treating a brain disease and a neuronal migration promoter. The present application is based on Japanese Patent Application No. 2022-144880, filed on September 12, 2022, the contents of which are herein incorporated by reference.

### [Background Art]

In order to fundamentally treat a brain disease such as cerebral infarction, a technology is necessary for regenerating lost nerve cells to restore a brain function. As such a technology, for example, a method has been known that includes transplanting cells derived from iPS cells as shown in PTL 1. In addition, as shown in PTL 2, a method has been known that promotes regeneration by inducing nerve cells produced from endogenous neural stem cells to an injury site. In PTL 2, migration of neurons (nerve cells) is promoted in damaged tissue generated on the surface layer of the brain by using a sponge-like material fixed or coated with N-cadherin or the like.

### [Citation List]

### [Patent Literature]

[PTL 1] WO 2013/069661 A
[PTL 2] JP 2020-018796 A

### [Summary of Invention]

### [Technical Problem]

When nerve cells or the like produced from iPS cells are transplanted to an injury site as described in PTL 1, there is a concern that invasion into the brain occurs and the transplanted cells form a tumor in the brain. In addition, a surgically invasive procedure is required to transplant an artificial scaffold such as a biomaterial as disclosed in PTL 2 in order to induce nerve cells produced from endogenous neural stem cells to an injury site. Therefore, there is room for improvement in clinical use as regenerative medicine for brain diseases. For this reason, other technologies capable of promoting migration and regeneration of neurons have been sought. Then, the present inventors have intensively studied and invented another technology capable of promoting migration and regeneration of neurons.

### [Solution to Problem]

The present invention can be implemented in the following aspects.

(1) One aspect of the present invention provides a pharmaceutical composition for treating a brain disease. This pharmaceutical composition comprises a neuraminidase inhibitor. The pharmaceutical composition of this aspect may be used to treat a brain disease because the inclusion of a neuraminidase inhibitor can promote the migration of neurons.
(2) The pharmaceutical composition according to (1), wherein the neuraminidase inhibitor may inhibit at least one of Neu1 or Neu4. The pharmaceutical composition of this aspect can effectively promote the migration of neurons because the neuraminidase inhibitor inhibits at least one of Neu1 or Neu4.
(3) The pharmaceutical composition according to the above (1) or (2), wherein administration starts before a lapse of 10 days from an onset of the brain disease, and the pharmaceutical composition may be administered daily. The pharmaceutical composition of this aspect can effectively promote the migration of neurons because the administration is started before a lapse of 10 days from an onset of the brain disease and the pharmaceutical composition is administered daily.
(4) The pharmaceutical composition according to (3), wherein the pharmaceutical composition may be administered for 10 days or longer. The pharmaceutical composition of this aspect can more effectively promote the migration of neurons because it is administered daily for a period of 10 days or longer.
(5) The pharmaceutical composition according to any one of the above (1) to (4), wherein the pharmaceutical composition may be administered in a form of an injection. The pharmaceutical composition of this aspect can reduce surgical invasiveness because it is administered in the form of an injection.
(6) Another aspect of the present invention provides a neuronal migration promoter. This neuronal migration promoter comprises a neuraminidase inhibitor. The neuronal migration promoter of this aspect may comprise a neuraminidase inhibitor to promote the migration of neurons.
(7) The neuronal migration promoter according to (6), wherein the neuraminidase inhibitor may inhibit at least one of Neu1 or Neu4. The neuronal migration promoter of this aspect can effectively promote the migration of neurons because the neuraminidase inhibitor inhibits at least one of Neu1 or Neu4.
(8) Another aspect of the present invention provides a method of treating a brain disease. This method comprises a step of administering a neuraminidase inhibitor to a brain disease patient. The method of this aspect includes the step of administering a neuraminidase inhibitor to a brain disease patient, thereby capable of promoting the migration of neurons. As a result, it can be used for treatment of the brain disease.
(9) Another aspect of the present invention provides a method of promoting migration of neurons. This method comprises a step of bringing a neuraminidase inhibitor into contact with neurons. The method of this aspect includes the step of bringing a neuraminidase inhibitor into contact with neurons, thereby capable of promoting the migration of neurons.
(10) Another aspect of the present invention provides use of a neuraminidase inhibitor for treatment of a brain disease.

Note that the present disclosure can be realized in various forms, and can be realized, for example, in the form of a migration promoter for new neurons, a kit for promoting migration of neurons, a kit including a pharmaceutical composition, a method for producing a neuronal migration promoter, a method for producing a pharmaceutical composition, a method of promoting migration of new neurons, a method for assisting in regeneration of damaged tissue in the brain, a method of treating a brain disease, use as a neuronal migration promoter, use for promoting migration of neurons, use of a neuraminidase inhibitor for the manufacture of a neuronal migration promoter, use of a neuraminidase inhibitor for the manufacture of a therapeutic agent for a brain disease, or the like.

### [Brief Description of Drawings]

[Fig. 1] A schematic diagram illustrating how a neuraminidase cleaves PSA-NCAM.
[Fig. 2] A flowchart illustrating a step in a method of treating a brain disease.
[Fig. 3] A flowchart illustrating a step in a method of promoting migration of neurons.
[Fig. 4] An explanatory view showing image data of SBF-SEM in Experiment 1.
[Fig. 5] A graph for explaining the proportion of cell adhesion as a result of Experiment 1.
[Fig. 6] An explanatory view showing microscopic images in Experiment 2.
[Fig. 7] A graph showing fluorescence intensity of PSA-NCAM in Experiment 2.
[Fig. 8] An explanatory view showing microscopic images in Experiment 3.
[Fig. 9] A graph for explaining the proportion of cell adhesion as a result of Experiment 3.
[Fig. 10] Graphs showing the expression levels of Neu1 to Neu4 in a normal brain and an injured brain.
[Fig. 11] A graph showing and comparing the expression levels of Neu1 to Neu4 in an injured brain.
[Fig. 12] A schematic diagram showing a brain injury site of a mouse in Experiment 5.
[Fig. 13] An explanatory view showing microscopic images in Experiment 5.
[Fig. 14] Graphs showing signal intensities in Experiment 5.
[Fig. 15] An explanatory view showing microscopic images in Experiment 6.
[Fig. 16] Graphs showing signal intensities in Experiment 6.
[Fig. 17] An explanatory view showing microscopic images in Experiment 7.
[Fig. 18] A graph showing the number of double-positive cells in Experiment 7.
[Fig. 19] A photo illustrating how FFT looks.
[Fig. 20] A graph showing the results of Experiment 8.
[Fig. 21] A schematic diagram illustrating brain areas used for quantification of sialic acid in Experiment 9.
[Fig. 22] Graphs showing the results of sialic acid quantification in Experiment 9.
[Fig. 23] A scheme illustrating an outline of Experiment 10.
[Fig. 24] An explanatory view showing representative microscopic images in Experiment 10.
[Fig. 25] A graph showing PSA signal intensities in Experiment 10.
[Fig. 26] An explanatory view showing a result of time-lapse imaging in Experiment 10.
[Fig. 27] A graph showing the migration speed of neurons in Experiment 10.
[Fig. 28] An explanatory view showing representative microscopic images in Experiment 11.
[Fig. 29] A graph showing the number of double-positive cells in Experiment 11.
[Fig. 30] Graphs showing the results of Experiment 12.
[Fig. 31] An explanatory view showing representative microscopic images in Experiment 13.
[Fig. 32] A graph showing PSA-NCAM fluorescence intensity in Experiment 13.
[Fig. 33] A graph showing a distance from an injury site in Experiment 13.

### [Description of Embodiments]

An embodiment of the present disclosure provides a neuronal migration promoter. This neuronal migration promoter comprises a neuraminidase inhibitor.

The neurons are not particularly limited, and examples thereof include new neurons produced from endogenous neural stem cells. Examples of the other neurons include neurons induced and differentiated from pluripotent stem cells such as iPS cells (induced pluripotent stem cells) and ES cells (embryonic stem cells). The subject is not particularly limited, and examples thereof include mammals including humans, and non-human mammals such as monkeys, mice, rats, rabbits, dogs, cats, sheep, goats, horses, cows, and pigs.

Neuraminidase is an enzyme that cleaves polysialic acid (PSA). The neuraminidase known includes an exo type (enzyme number EC 3.2.1.18) and an endo type (enzyme number EC 3.2.1.129). Note that the neuraminidase is also called sialidase. Four known subtypes of neuraminidases derived from mammals include Neu1, Neu2, Neu3, and Neu4. Reference Sequence RNA of human-derived Neu1 is NM_000434. Reference Sequence RNA of human-derived Neu2 is NM_005383. Reference Sequence RNA of human-derived Neu3 is NM_006656. Reference Sequence RNA of human-derived Neu4 is NM_080741.

The neuraminidase inhibitor may include a substance that inhibits the activity of neuraminidase and a substance that inhibits the expression of neuraminidase. The neuraminidase inhibitor is not particularly limited, and examples thereof include 2,3-dehydro-2-deoxy-N-acetylneuraminic acid (DANA), Zanamivir, Oseltamivir, Peramivir, Laninamivir, and siRNA and/or miRNA that inhibits expression of neuraminidase. As shown in Examples described later, it is preferable that the neuraminidase inhibitor inhibits at least one of Neu1 or Neu4. Therefore, it is preferable to contain a human Neul-specific inhibitor (e.g., Magesh *et al.,* 2008) or a Neu4-specific inhibitor. Among the clinical drugs, Zanamivir is preferable from the viewpoint that an inhibitory effect has been reported for at least Neu2, Neu3, and Neu4 among human neuraminidases.

The mechanism by which the migration of neurons is promoted by including the neuraminidase inhibitor is not clear. The following possible mechanism is assumed from the results shown in Examples described later.

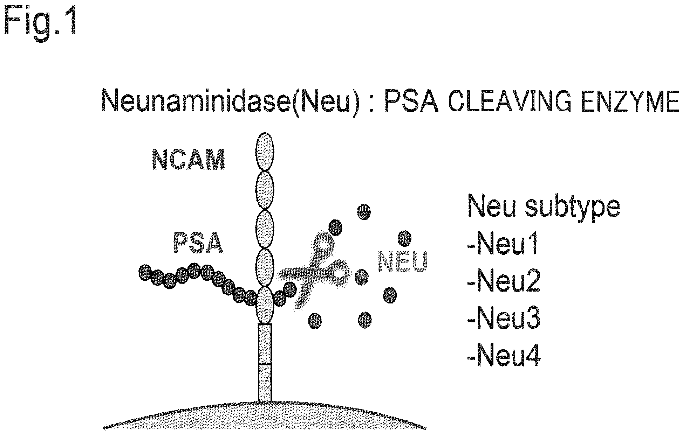

Fig. 1 is a schematic diagram illustrating how a neuraminidase cleaves PSA-NCAM. Note that a neural cell adhesion molecule (NCAM) is a cell adhesion molecule. It is considered that polysialic acid produces an exclusive space by having a bulky structure. Here, the present inventors have considered that a phenomenon occurs in which sugar chain polysialic acid on the nerve cell surface is cleaved by neuraminidase through a process in which nerve cells produced by neural stem cells present in the subventricular zone migrate to an injury site after brain injury. It is assumed that cleavage of the sugar chain polysialic acid causes excessive cell adhesion between nerve cells and suppresses migration of the nerve cells. For this reason, the present inventors have thought that the migration of nerve cells can be promoted by suppressing the cleavage of the sugar chain polysialic acid by the neuraminidase inhibitor and consequently suppressing excessive cell adhesion. In addition, a large number of nerve cells can migrate to the injury site. As a result, it is expected that more immature nerve cells reach the injury site and differentiate and mature into nerve cells. As a result, since the regeneration of nerve cells is promoted, it is considered that it is also possible to restore the brain function and the motor function deteriorated by the brain disease.

The neuronal migration promoter of this embodiment may be used for treatment or prevention of a disease or condition. Examples of the disease or condition include, but are not particularly limited to, hypoxic-ischemic encephalopathy, cerebral infarction, cerebral hemorrhage, cerebrovascular disorder, central nervous system disease, Alzheimer's disease, cognitive impairment, or trauma. Thus, another aspect of the present disclosure provides a pharmaceutical composition for treating a brain disease. This pharmaceutical composition comprises a neuraminidase inhibitor.

The neuronal migration promoter and the pharmaceutical composition may comprise a pharmaceutically acceptable carrier. The term "pharmaceutically acceptable carrier" herein refers to an additive usually used in the art of formulation. Examples of such a carrier include, but are not particularly limited to, a solvent, an excipient, a filler, an emulsifier, a fluidity modifier, a lubricant, and/or human serum albumin. The solvent may be, for example, either water or another pharmaceutically acceptable aqueous solution, or a pharmaceutically acceptable organic solvent. Examples of the aqueous solution include, but are not particularly limited to, physiological saline, an isotonic solution containing glucose and other auxiliary agents, a phosphate buffer, or a sodium acetate buffer. Examples of the auxiliary agent include, but are not particularly limited to, D-sorbitol, D-mannose, D-mannitol, sodium chloride, as well as nonionic surfactants at a low concentration, or polyoxyethylene sorbitan fatty acid esters.

Examples of the excipient include, but are not particularly limited to, sugars such as monosaccharides, disaccharides, cyclodextrins and polysaccharides, metal salts, citric acid, tartaric acid, glycine, polyethylene glycol, Pluronic (registered trademark), kaolin, silicic acid, or combinations thereof. Examples of the filler include, but are not particularly limited to, petrolatum, the above sugar, and/or calcium phosphate. Examples of the emulsifier include, but are not particularly limited to, sorbitan fatty acid ester, glycerin fatty acid ester, sucrose fatty acid ester, or propylene glycol fatty acid ester. Examples of the fluidity modifier and the lubricant include, but are not particularly limited to, silicate, talc, stearate, or polyethylene glycol. In addition to the above, optionally included are, if appropriate, a solubilizer, a suspending agent, a diluent, a dispersant, a surfactant, a soothing agent, a stabilizer, an absorption promoting agent, an extender, a humectant, a moisturizer, a wetting agent, an adsorbent, a flavoring agent, a disintegration inhibitor, a coating agent, a coloring agent, a preservative, an antiseptic, an antioxidant, a perfume, a flavor agent, a sweetening agent, a buffering agent, an isotonizing agent, and/or the like, which are usually used in pharmaceuticals.

The dosage form of the neuronal migration promoter according to this embodiment is not particularly limited, but is preferably a form that can be delivered to a target site without inactivating properties in the brain of the subject. Examples of the dosage form of the neuronal migration promoter include, but are not particularly limited to, a liquid preparation, a powder preparation, granules, or a tablet. The dosage form of the neuronal migration promoter is preferably a liquid preparation from the viewpoint of facilitating delivery to a target site in the brain of the subject. Examples of the liquid preparation include, but are not particularly limited to, an injectable form such as an injection and an instillation. Preferable examples of the injection include, but are not particularly limited to, an arterial injection or an intramuscular injection from the viewpoint of easily delivering the neuronal migration promoter to a target site. According to such a form, it is possible to reduce surgical invasiveness. Further, such a form can readily promote the migration of neurons in the brain of the subject. In addition, the migration of neurons can be promoted by a minimally invasive and safe technique as compared with, for instance, local transplantation of cells or scaffold materials. In addition, it is possible to regenerate nerve cells against a wide range of injuries in the brain as compared with, for instance, local transplantation of cells or scaffold materials. In addition, the neuronal migration promoter may be directly administered into the brain, or may be impregnated in a scaffold material or the like and transplanted into the brain. In addition, the neuronal migration promoter may have a sustained release property, and may have a form in which the neuraminidase inhibitor is subjected to a sustained release over a period of several hours, several days, several weeks, or the like by staying at a target site in the brain of the subject. The neuronal migration promoter may be formulated, if appropriate, in combination with the above mentioned stabilizer, pH adjuster, excipient, or the like, or may be combined with an instrument, such as a syringe for use in administration, to be provided as a kit.

The timing and dose of administration of the neuronal migration promoter and the pharmaceutical composition in this embodiment may be appropriately determined according to the subject for administration, the disease, or the condition. The administration timing is not particularly limited, but for example, it is desirable to perform continuous administration for a period in which migration of neurons is desirably promoted. The continuous administration is preferably administration once or more every three days, more preferably administration once or more every two days, and still more preferably administration once a day, that is, daily. In addition, the start timing of administration is not particularly limited, but is preferably, for example, a timing at which the neurons start migrating or a timing before the neurons start migrating. The start timing of administration is not particularly limited, but for example, from the viewpoint of effectively promoting migration of neurons, it is preferably before the lapse of 20 days from the onset of the brain disease, more preferably before the lapse of 15 days, still more preferably before the lapse of 12 days, still even more preferably before the lapse of 10 days, and particularly preferably before the lapse of 8 days. In addition, the start timing of administration is not particularly limited, but for example, from the viewpoint of effectively promoting migration of neurons, it is preferably after the lapse of three days from the onset of the brain disease, more preferably after the lapse of five days, and still more preferably after the lapse of seven days. The administration period is not particularly limited, but for example, from the viewpoint of effectively promoting migration of neurons, the administration period is preferably 5 days or longer, more preferably 7 days or longer, still more preferably 10 days or longer, still even more preferably 12 days or longer, and particularly preferably 14 days or longer. Preferred is daily administration for 10 days or longer following the start of administration after the lapse of 3 days and before the lapse of 10 days from the onset of the brain disease. More preferred is administration for 14 days or longer following the start of administration after the lapse of 5 days and before the lapse of 8 days from the onset of the brain disease. For example, the administration may be started on day 7 after the onset of the brain disease and continue for 14 days. The dose varies depending on the severity of the brain disease, the sex, age, weight, and the like of the patient, and may be, for example, 1 to 90 mg/kg body weight/day of the neuraminidase inhibitor.

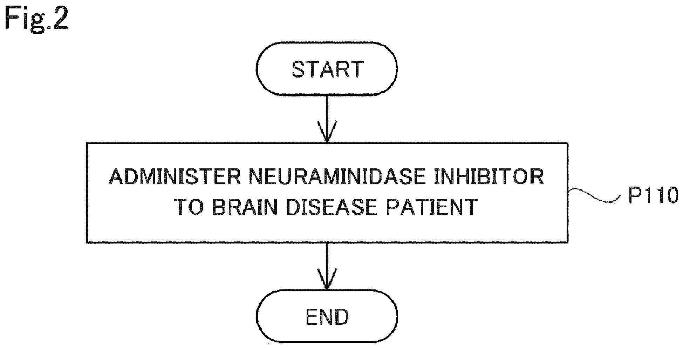

Another aspect of the present disclosure provides a method of treating a brain disease. Fig. 2 is a flowchart illustrating a step in the method of treating a brain disease. This method comprises the step (P110) of administering a neuraminidase inhibitor to a brain disease patient. This step may be performed according to the above-described administration timing and dose. In addition, the method of treating a brain disease may further include, before the step of administering a neuraminidase inhibitor to a brain disease patient, a step of determining a dose of the neuraminidase inhibitor. In this step, for example, the dose may be determined according to the severity of the brain disease or the sex, age, and weight of the patient. In addition, the method of treating a brain disease may further include, after the step of administering a neuraminidase inhibitor to a brain disease patient, a step of checking efficacy caused by the administration. In this step, for example, the efficacy may be checked by a behavior test.

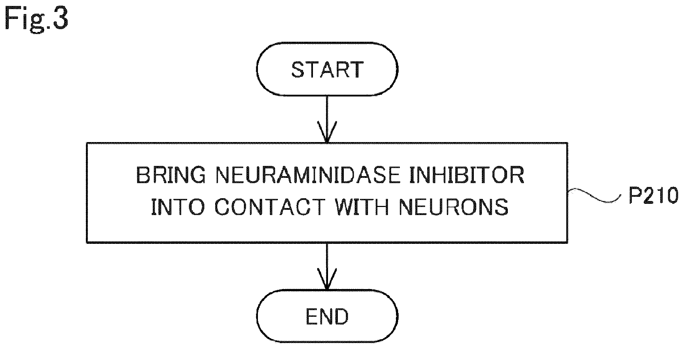

Another aspect of the present disclosure provides a method of promoting migration of neurons. Fig. 3 is a flowchart illustrating a step in the method of promoting migration of neurons. This method comprises the step (P210) of bringing a neuraminidase inhibitor into contact with neurons. The step of bringing a neuraminidase inhibitor into contact with neurons may be realized by administering the neuraminidase inhibitor to a subject. In addition, this administration may be performed according to the above-described administration timing and dose. Note that the step for bringing a neuraminidase inhibitor into contact with neurons need not include medical practice for humans.

### [Examples]

Hereinafter, the present invention will be described more specifically with reference to Examples. The present invention, however, is not limited to the following Examples.

### 1. Materials and methods

### (1) Materials

Wild-type (WT) male and female C57BL/6J mice were purchased from Japan SLC, Inc. (RRID: IMSR_JAX: 000664). Dcx-GFP mice were obtained by the method described in Gong et al., Nature 425, p. 917-925 (2003). Nestin-CreERT2; Neuron-specific enolase (NSE)-diphtheria toxin fragment A (DTA) mice were obtained by the method described in Nat. Neurosci. 11, 1153-61 (2008). Common marmoset (*Cαllithrix jacchus*) individuals were obtained from a mating pair of a breeding animal colony. Tamoxifen was administered by the method described in Nat. Neurosci. 11, 1153-61 (2008).

### (2) To induce cerebral cortex injury

As the injured brain, an injury of the cerebral cortex of a mouse was artificially induced. The cerebral cortex injury was induced by improving a method for preparing an ischemic brain injury model by light stimulation (Photothrombosis) reported by Watson *et al.* (Watson *et al.,* 1985) , as described below. First, 8 to 12-week-old mice were each anesthetized by administering an oxygen/isoflurane gas mixture (97.5/2.5%) while using an inhalation mask and then placed on a heating bed at 37°C. The scalp was dissected to expose the skull surface, and the bregma and a target site appeared. A 8-mm-diameter optical fiber cable connected to a light source (MSG6-1100S, manufactured by MORITEX Corporation) for light irradiation was positioned on the skull and fixed 1 mm frontal and 2.7 mm lateral to the bregma. Cerebral cortex ischemic injury was induced by activating light-sensitive rose bengal dye (30 mg/kg, 330000-1G, manufactured by Sigma-Aldrich) in 0.9% NaCl solution. The rose bengal was infused intravenously. The skull was irradiated for 10 minutes using MHAA-100W-100V (manufactured by MORITEX Corporation) under conditions at a wavelength of 533 nm and 150 mW. For common marmoset individuals, a modified method for preparing an ischemic brain injury model by light stimulation (Photothrombosis) was used in accordance with the description of PLoS One. 8, e60037 (2013). Cerebral ischemia in each common marmoset (3 months old), namely intravascular thrombosis was induced by intravenous injection of rose bengal (30 mg/kg) under deep anesthesia and irradiation with green light (533 nm; a metal halide lamp, PCS-MH357RC, manufactured by NIPPON P·I CO., LTD.) for 5 to 10 minutes. All surgeries were performed under general anesthesia induced by intramuscular injections of medetomidine (0.04 mg/kg), midazolam (0.4 mg/kg), and butorphanol (0.4 mg/kg), maintained with isoflurane, and antagonized with atipamezole (0.15 mg/kg). The scalp was dissected to expose the skull surface, and the bregma and a target site appeared after washing. A 12-mm-diameter optical fiber cable connected to a light source (PLG-1-1000-3R-UX350, manufactured by NIPPON P·I CO., LTD.) for light irradiation was positioned on the skull and fixed 5 mm frontal and 6 mm lateral to the bregma.

### (3) Serial block-face scanning electron microscopy (SBF-SEM)

Sample preparation, observation by SBF-SEM (serial block-face scanning electron microscopy), and analysis of acquired data were performed in accordance with existing methods with some modifications (Kaneko *et al,* 2018; Nguyen *et al,* 2016; Sawada *et al,* 2018; Thai *et al,* 2016). Brains of adult wild-type mice were perfused in 0.1 M phosphate buffer (pH 7.4) containing 2.5% glutaraldehyde (GA) and 2% PFA at 4°C and further fixed using the same fixative overnight at 4°C. A fixed RMS or near-brain injury tissue was treated with 2% OsO₄ and 1.5% potassium hexacyanoferrate in PBS for 1 hour at 4°C. Thereafter, each tissue was treated in 1% thiocarbohydrazine at room temperature for 20 minutes, treated in 2% OsO₄ aqueous solution at room temperature for 30 minutes, and then treated in a lead aspartate aqueous solution at 65°C for 30 minutes. After that, the RMS tissue was dehydrated by ethanol serial dilution, treated using dehydrated acetone, and then embedded in Durcupan resin containing Ketjen black powder at 95°C for 3 hours for polymerization. The RMS tissue in the normal brain and the RMS tissue in the injured brain were observed by SBF-SEM using a Merlin and Sigma scanning electron microscope (manufactured by Carl Zeiss) equipped with a 3View in-chamber ultramicrotome system (manufactured by Gatan). A serial image was created in 80 nm steps with a width of 40.96 × 40.96 µm (5.0 nm/pixel) and a depth of 80 µm or more. The serial image was also processed with FIJI. Cell membranes, nuclei, and distal spheres were segmented using a Microscopy Image Browser (Belevich *et al.,* 2016). Manual segmentation was performed on all slices. Migrating new neurons and their intracellular organs were three-dimensionally reconstituted by a known method using Amira software (manufactured by Maxnet) (Kaneko *et al.,* 2018; Sawada *et al.,* 2018; Matsumoto *et al.,* 2019).

### (4) Immunohistochemistry and image acquisition

Immunohistochemistry of mouse brain sections was performed according to known methods (Ota *et al.,* 2014; Sawada *et al.,* 2018). More specifically, brains of adult mice were perfused in 0.1M phosphate buffer (PB) containing 4% paraformaldehyde (PFA) and further fixed using the same fixative overnight at 4°C. Floating coronal and sagittal sections of 50-µm thickness were prepared with a vibratome (VT-1200S, manufactured by Leica) and incubated for 30 min at room temperature in PBS containing 10% normal donkey serum (NDS) and 0.2% Triton X-100 as a blocking solution. These sections were incubated with primary antibodies in a blocking solution overnight at 4°C, followed by AlexaFluor488/568/647 labeled secondary antibodies (1:1,000, manufactured by Invitrogen) in a blocking solution for 2 hours at room temperature. The primary antibodies used were the following antibodies: rabbit anti-doublecortin (Dcx) (1:1,000, 4604S, manufactured by Cell Signaling Technology; RRID: AB_10693771), rabbit anti-DsRed (1:1,000, 632496, manufactured by Clontech; RRID: AB_10013483), mouse IgM anti-polysialated neuronal adhesion molecule (PSA-NCAM) (1:1000, provided by Honorary Professor Tatsunori Seki, Tokyo Medical University) (Seki and Arai, 1991), and rabbit anti-NeuN (1:1000, ab177487, manufactured by Abcam). Immunohistochemistry of common marmoset brain sections was performed according to the method described in Cereb. Cortex. 30, 4092-4109 (2020). More specifically, adult brains were perfused in 0.1 M phosphate buffer (PB) containing 4% paraformaldehyde (PFA) and further fixed using the same fixative overnight at 4°C. Coronal and sagittal sections having a thickness of 60 µm were prepared using a vibratome (VT-1200S, manufactured by Leica), and incubated in a blocking solution at room temperature for 1 hour. These sections were incubated with primary antibodies in a blocking solution overnight at 4°C, followed by AlexaFluor488/647 labeled secondary antibodies (1:1,000, manufactured by Invitrogen) in a blocking solution for 3 hours at room temperature. The primary antibodies used were guinea pig anti-Dcx (1:400, AB2253, manufactured by Millipore; RRID: AB_1586992) and mouse IgM anti-PSA-NCAM (1:400) antibodies.

Images were acquired by scanning at 2 µm intervals while using an LSM 700 confocal laser scanning microscope (manufactured by Carl Zeiss) equipped with a 20× objective lens. The fluorescence intensity of PSA-NCAM in Dcx+ cells (Dcx positive cells) was quantified using ZEN software (manufactured by Carl Zeiss). Dcx-positive cells or Dcx-positive and NeuN-positive cells in the injured brain were counted every 6 coronal sections with a 50-µm thickness. The total number per hemisphere was estimated by multiplying the sum by 6.

### (5) Real-time PCR

To examine the mRNA levels of Neu1 to Neu4 in normal and injured brains, normal brains or brains at day 7 after injury of wild-type mice were rapidly removed from the skull under deep anesthesia, and the cortex (Ctx), corpus callosum (CC) and striatum (St) in the normal or injured brains were detached. In the following description, the cortex and the corpus callosum are also collectively referred to as "Ctx, CC". Total RNA was extracted from each collected sample. For the extraction, an RNA extraction kit (CellAmp Direct RNA prep kit, manufactured by Takara) was used. In order to examine the mRNA levels of Neu1 to Neu4 in the cortex, corpus callosum, striatum of the normal or injured brain, total RNA was extracted from each tissue by using a TRIzol reagent (manufactured by Invitrogen). cDNA was synthesized using SuperScript IV Reverse Transcriptase (manufactured by Invitrogen). Quantitative SYBR Green real-time PCR was performed according to a known method using an ABI 7500 Fast Real-Time PCR apparatus (manufactured by Applied Biosystems) (LS Zheng *et al,* 2015). The following primers were used for real-time PCR. Specifically used were: Neu1 forward primer 5'-TGCCAGCCCTACGAGCTT-3' (SEQ ID NO: 1) and Neu1 reverse primer 5'-TGGTTCCGGCGTTGAT-3' (SEQ ID NO: 2) for amplifying the 52bp Neu1 product; Neu2 forward primer 5'-CCCTGGCGTGTATCAGAA-3' (SEQ ID NO: 3) and Neu2 reverse primer 5'-AGCACAGCCGTGTGACATTAAC-3' (SEQ ID NO: 4) for amplifying the 61bp Neu2 product; Neu3 forward primer 5'-GGCTGGACGGCTGGTA-3' (SEQ ID NO: 5) and Neu3 reverse primer 5'-TCGAAATCGGCTTGGTGTTC-3' (SEQ ID NO: 6) for amplifying the 60bp Neu3 product; Neu4 forward primer 5'-AAGGCACGTCCTTCCTACCA-3' (SEQ ID NO: 7) and Neu4 reverse primer 5'-GCAACCACGAGCCGTCTCT-3' (SEQ ID NO: 8) for amplifying the 61bp Neu4 product; and GAPDH forward primer 5'-CATGGCCTTCCGTGTTCCTA-3' (SEQ ID NO: 9) and GAPDH reverse primer 5'-CACGTCAGATCCA-3' (SEQ ID NO: 10) for amplifying the 55bp GAPDH product as an endogenous control.

### (6) Viral Vectors and Plasmids

A Neu1 knockdown plasmid and a Neu4 knockdown plasmid were constructed according to a known method (Ota *et al*, 2014). More specifically, target sequences of mouse Neu1, Neu4, and LacZ (comparative example) genes were each inserted into a modified Block-iT Pol II miR RNAi expression vector containing DsRed-express. The following pCSII lentiviral expression vectors were constructed using the Gateway system (manufactured by Invitrogen) (CSII-EF-DsRed-miR-Neu1, CSII-EF-DsRed-miR-Neu4, CSII-EF-DsRed-miR-LacZ miRNA). Knockdown (KD) efficiency in these vectors was assessed by Western blotting. Note that the sequence of each miRNA used was as follows: miRNA insert for knockdown of Neul: TGCTGATAAGGAACACTATCCCTGTGGTTTTGGCCACTGACTGACCACAGGGAGTGTT CCTTAT (SEQ ID NO: 11), and miRNA insert for knockdown of Neu4: TGCTGTTCATAGGAAGTCCTGGTCCCGTTTTGGCCACTGACTGACGGGACCAGCTTCCT ATGAA (SEQ ID NO: 12).

To generate lentiviral particles, the lentiviral and packaging vectors (pCAG-HIVgp and pCMV-VSV-G-RSV-Rev) were transfected into HEK293T cells. Three days after the transfection, the cells were centrifuged using a MX-307 micro cooling centrifuge (manufactured by Tomy) at 8000 rpm and 4°C for 16 hours, and the culture supernatant was thus concentrated. Then, 2 µL of the lentiviral suspension was injected stereotactically around the area of injury (1.8, 1.6, 1.4, 1.2, 1.0, 0.8, 0.5 mm anterior, lambda lateral, 1.5-2.0 mm deep) in mice 7 days post-brain injury (dpi).

### (7) Co-culture of V-SVZ-derived new neurons with BV2 cells

*In vitro* culture and time-lapse imaging of new neurons derived from V-SVZ were performed according to the method described in J. Neurosci. 39, 9967-9988 (2019). More specifically, V-SVZ tissues of P0-P3 female and male wild-type mice were dissected and dissociated with trypsin-EDTA (manufactured by Invitrogen). Cells were washed with L-15 medium (manufactured by Invitrogen) containing 40 µg/ml DNase I (manufactured by Roche Diagnostics) and transfected with a plasmid pCAGGS-EGFP using the Amaxa Nucleofector IV system (manufactured by Lonza Walkersville). Transfected cells were collected in RPMI-1640 medium (manufactured by Thermo Fisher Scientific) and re-aggregated. These cell aggregates were placed on a monolayer of BV2 cells (provided by Dr. Takatoshi Ueki, Nagoya City University) embedded in 60% Matrigel (manufactured by BD Biosciences), and cultured in Neurobasal medium containing 2% NeuroBrew-21 (manufactured by Invitrogen), 2 mM L-glutamine (manufactured by Invitrogen), and 50 U/ml penicillin-streptomycin (manufactured by Invitrogen) for 2 days, followed by time-lapse imaging. The cells were treated with lipopolysaccharide (LPS; 100 ng/ml to 1 µg/ml) in combination with or without a neuraminidase inhibitor (DANA or Zanamivir; final concentration: 1mM) in accordance with the method described in J. Biol. Chem. 290, 13202-14 (2015). Time-lapse imaging of randomly selected EGFP-expressing new neurons was performed using a fluorescence microscope (manufactured by KEYENCE) and a 20× objective lens.

### (8) Method of EndoN treatment and method of administering neuraminidase Inhibitor

First, 1 µl of PBS or EndoN (10 units/µl) was stereotactically injected into the lateral ventricle (-0.2 mm anterior, 0.6 mm lateral, 2.0 mm deep to the bregma). For inhibition of neuraminidase, DANA (N-Acetyl-2,3-didehydro-2-deoxyneuraminic acid) (50 mg/kg), Zanamivir (50 mg/kg), or PBS (control) was intraperitoneally injected daily at 7-21 dpi for mice or at 7-28 dpi for common marmosets.

### (9) To quantify sialic acid

Brain samples from dissected mice were homogenized in PBS containing 1% Triton X-100, a protease inhibitor cocktail (1 µg/mL aprotinin, 1 µg/mL leupeptin, 1 µg/mL pepstatin, 2 µg/mL antipane), and 5 mM ethylenediaminetetraacetic acid (EDTA), and incubated for 1 hour on ice. The resulting homogenates were centrifuged and the supernatant protein concentration was measured using a bicinchoninic acid (BCA) assay. Sialic acid (Sia) in each sample was quantified using a DMB derivatization method. More particularly, 1 µl of brain homogenate was hydrolyzed by incubation in 0.2 M trifluoroacetic acid (TFA) at 80°C for 2 hours. For DMB derivatization, 20 µl of 0.01 M TFA and 20 µl of DMB solution were added to the dry sample, and the mixture was incubated at 50°C for 2 hours. For fluorescent HPLC analysis, samples were diluted 10 fold, injected from a JASCO Intelligent Inert Sampler (AS-4050) into a Handy ODS column (250 × 4.6 mm i.d.) (manufactured by Wako), and eluted with methanol/acetonitrile/water (7/9/84, v/v/v). The HPLC system was composed of a JASCO pump (PU-4180), a JASCO fluorescence detector (FP-2025 plus; excitation wavelength: 373 nm; emission wavelength: 448 nm), and a Chromato-PRO Integrator (manufactured by Run Time Corporation).

### 2. Experimental details and results

### <Experiment 1: To check morphology and cell adhesion of new neurons>

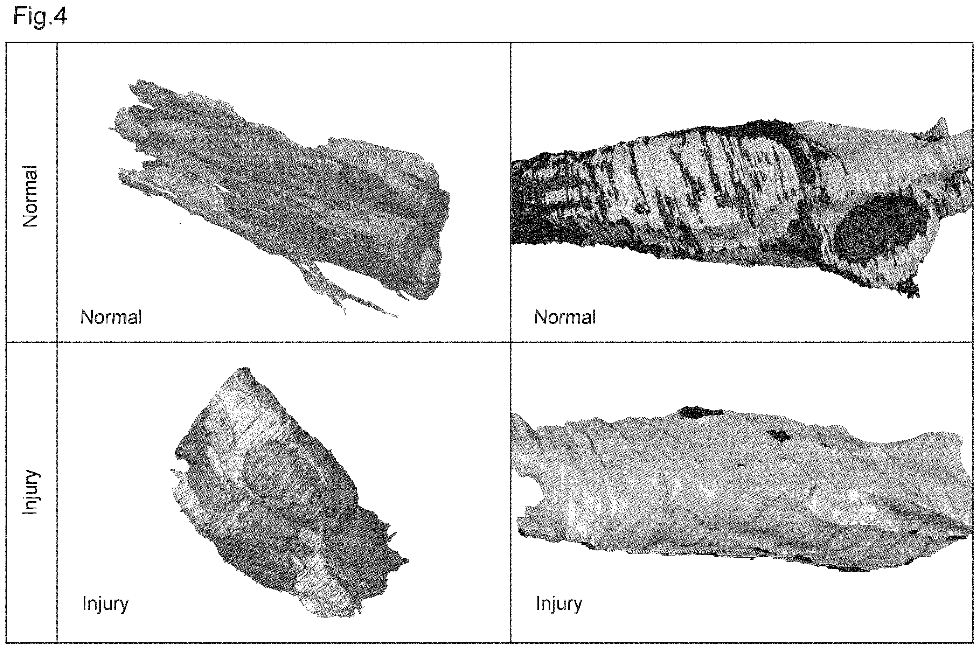

Morphology of a chain of new neurons and cell adhesion in a normal or injured brain were observed by SBF-SEM. In addition, the proportion of cell adhesion was quantified based on the results of SBF-SEM. Fig. 4 is an explanatory view showing image data of SBF-SEM in Experiment 1. The upper side of the drawing of Fig. 4 indicates a normal brain (Normal), and the lower side of the drawing indicates an injured brain (Injury). In addition, the left side of the drawing of Fig. 4 shows a chain-like population (chain) of cells, and each cell is color-coded. The right side of the drawing in Fig. 4 indicates the state of cell adhesion. The region indicated by light gray represents that new neurons adhere to each other, and the region indicated by dark gray represents an open extracellular space between neurons.

New neurons migrate while forming a chain in the brain. As illustrated on the left side of the drawing of Fig. 4, the chain in the normal brain shows that the orientation of cells is uniform, and each new neuron is in a smooth form. By contrast, in the chain in the injured brain, it can be seen that the orientation of the cells is non-uniform, and each new neuron is also in an irregular form.

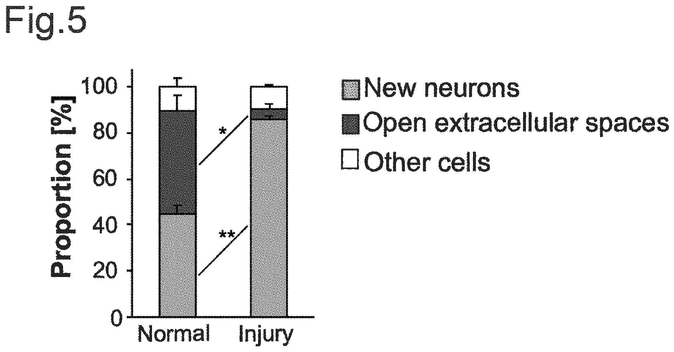

Fig. 5 is a graph for explaining the proportion of cell adhesion as a result of Experiment 1. In Fig. 5, similarly to the right side of the drawing of Fig. 4, a region in which new neurons adhere to each other (New neurons) is depicted in light gray, spaces between neurons are depicted in dark gray, and a region to which other cells adhere is depicted in white. The vertical axis in Fig. 5 represents the proportion, namely percentage (%), of the area of each region. As shown on the right side of the drawing of Fig. 4 and Fig. 5, in the injured brain, the space between neurons was significantly smaller and the proportion of the area of the region where the new neurons adhered to each other was significantly larger than those in the normal brain. The space between neurons is known as a characteristic of new neurons migrating as a population. For this reason, it has been suggested that the new neurons migrating in the injured brain had excessive cell adhesion as compared with the normal brain, and as a result, migration irregularity occurred.

### <Experiment 2: To compare PSA signal intensity of new neurons between normal brain and injured brain>

Polysialic acid (PSA) is present on the surface of a new neuron migrating in a normal brain. In addition, PSA is also widely used as a new neuron marker. However, details of PSA of new neurons migrating in an injured brain have not been revealed so far. Hence, the present inventors compared PSA signal intensity of new neurons between the normal brain and the injured brain>

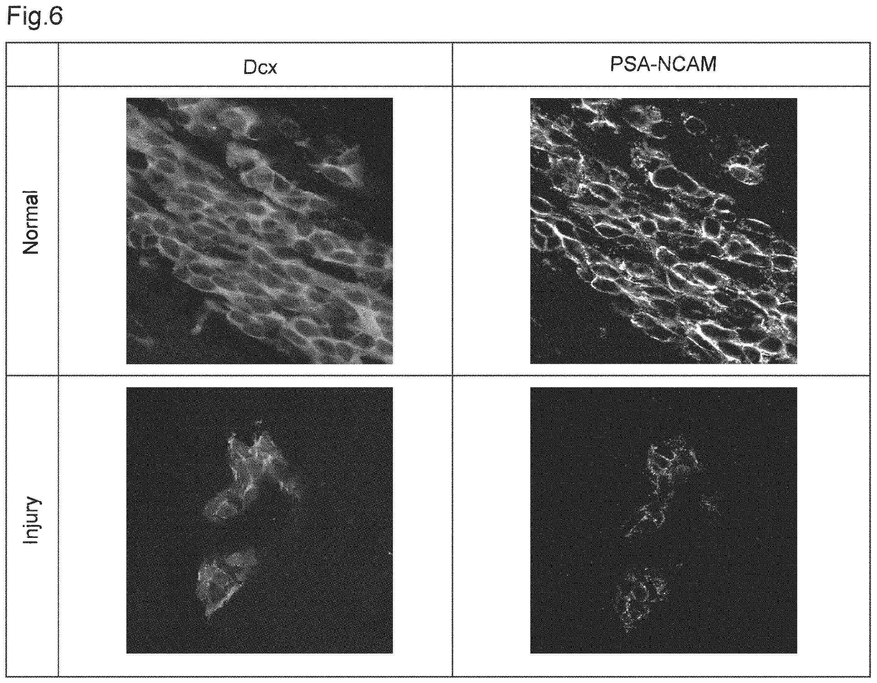

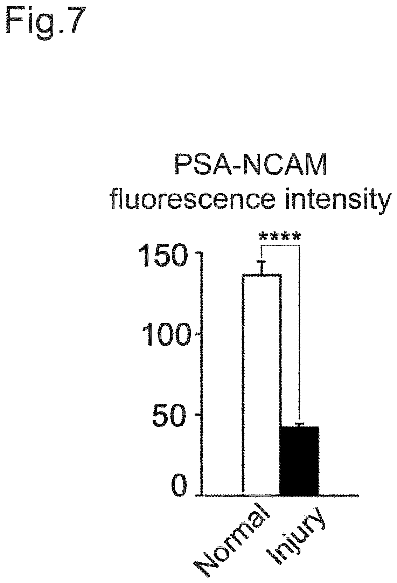

Fig. 6 is an explanatory view showing microscopic images in Experiment 2. The upper side of the drawing of Fig. 6 indicates a normal brain (Normal), and the lower side of the drawing indicates an injured brain (Injury). In addition, the left side of the drawing of Fig. 6 shows expression of Dcx, and the right side of the drawing of Fig. 6 shows expression of PSA-NCAM. Note that Dcx is a marker of immature neurons, and PSA-NCAM is a polysialylated neural adhesion molecule. Fig. 7 is a graph showing PSA-NCAM fluorescence intensity in Experiment 2. Fig. 7 shows the fluorescence intensity of PSA-NCAM in Dcx-positive cells.

The results shown in Figs. 6 and 7 have revealed the following. That is, it was found that the expression of PSA was also observed in new neurons of the injured brain, but the signal intensity was significantly lower than that of new neurons of the normal brain. This result suggests that a decrease in PSA level is involved in excessive adhesion between migrating new neurons in the injured brain.

### <Experiment 3: To check how administration of PSA-degrading enzyme affects interneuron adhesion>

In order to examine whether the presence of PSA affected the adhesion of new neurons, endoneuraminidaseN (endoN), which is a PSA-degrading enzyme, was administered into a normal brain, so that PSA on new neurons was removed. Specifically, 1 µL of PBS (phosphate-buffered saline, Comparative Example) or 1 µL of endoN (10 units/µL) was injected into the lateral ventricle of a Dcx-GFP mouse. After 3 days, fixation was performed, and observation by immunohistochemistry and observation by SBF-SEM were conducted.

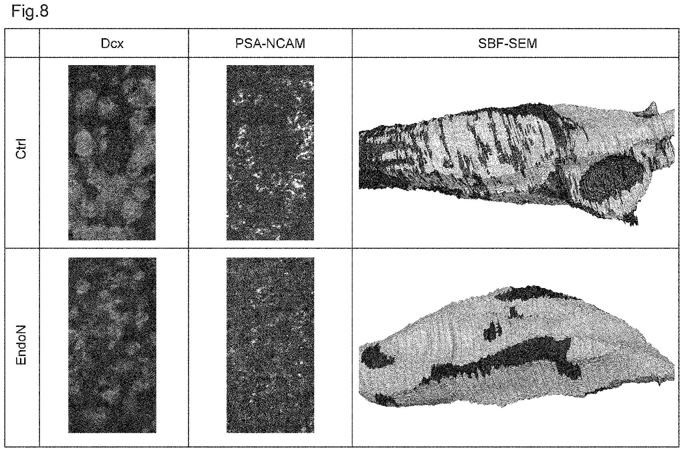

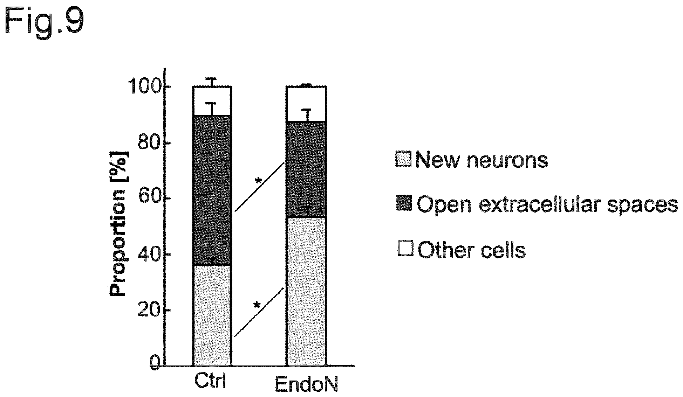

Fig. 8 is an explanatory view showing microscopic images in Experiment 3. The upper side of the drawing of Fig. 8 shows a Comparative Example (Ctrl), and the lower side of the drawing shows the endoN administration group (EndoN). In addition, the left side of the drawing of Fig. 8 shows expression of Dcx, the middle of the drawing shows expression of PSA-NCAM, and the right side of the drawing shows SBF-SEM images. In each SBF-SEM image, a region indicated by light gray represents that new neurons adhere to each other, and a region indicated by dark gray represents a space between neurons. Fig. 9 is a graph for explaining the proportion of cell adhesion as a result of Experiment 3. In Fig. 9, similarly to the right side of the drawing of Fig. 4 and the right side of the drawing of Fig. 8, a region in which new neurons adhere to each other is depicted in light gray, spaces between neurons are depicted in dark gray, and a region to which other cells adhere is depicted in white. The vertical axis in Fig. 9 represents the proportion, namely percentage (%), of the area of each region.

As shown in the middle of the drawing of Fig. 8, the signal intensity of PSA was significantly lower in the endoN administration group than that of the Comparative Example. As shown on the right side of the drawing of Fig. 8 and Fig. 9, cell adhesion was three-dimensionally observed by SBF-SEM. This revealed that in the endoN administration group, the proportion of spaces between neurons significantly decreased, and the proportion of adhesion between neurons increased. The results shown in Fig. 8 and Fig. 9 have suggested that in the injured brain, excessive cell adhesion was caused by a decrease in PSA.

### <Experiment 4: To examine possibility that neuraminidase is involved in decrease in PSA>

The possibility was examined as to whether neuraminidase (Neu), which is an enzyme that hydrolyzes sialic acid, was involved in the decrease in PSA of new neurons migrating in the injured brain. For normal and injured brains, the expression levels of Neu1 to Neu4 in the cerebral cortex (Ctx), corpus callosum (CC), and striatum (St) were quantified using real-time PCR.

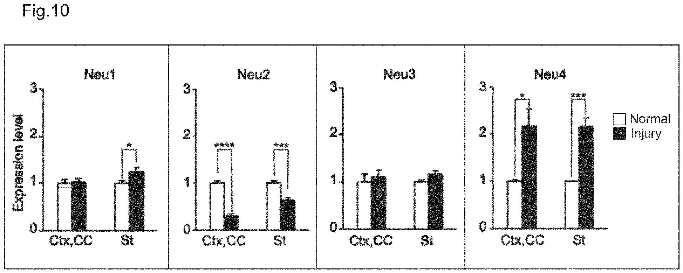

Fig. 10 provides graphs showing the expression levels of Neu1 to Neu4 in a normal brain and an injured brain. In the graphs of Fig. 10, the result in the normal brain is depicted in white, the result in the injured brain is depicted in black, and the expression levels in the cortex and the corpus callosum are collectively shown. The vertical axis in Fig. 10 indicates the relative expression level when the sum of the expression levels in the cortex and the corpus callosum of the normal brain is set to 1.

The results shown in Fig. 10 have revealed the following. That is, it was found that in the injured brain, the expression level of Neu1 in the striatum and the expression level of Neu4 in the entire region were higher than the expression levels in the normal brain. In particular, the expression level of Neu4 was increased by 2 times or more in the injured brain as compared with the normal brain.

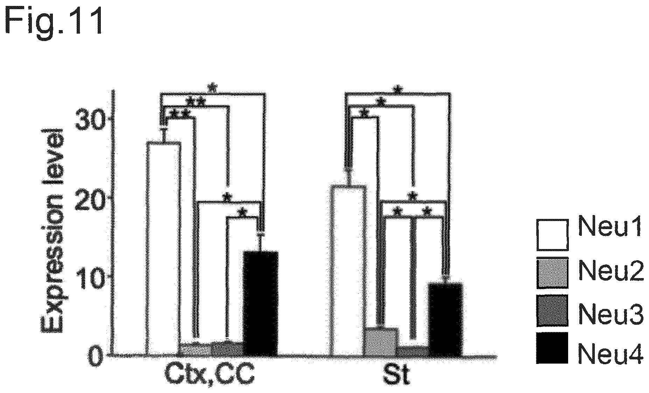

Fig. 11 is a graph showing and comparing the expression levels of Neu1 to Neu4 in an injured brain. In the graph of Fig. 11, the expression levels in the cortex and the corpus callosum are collectively shown, and the vertical axis of Fig. 11 shows the relative expression level when the expression level of Neu3 in the striatum, which expression level was the lowest, is set to 1.

The results shown in Fig. 11 have revealed the following. That is, the expression levels of Neu1 and Neu4 were high in any of the cortex, corpus callosum, and striatum. The results shown in Fig. 10 and Fig. 11 have suggested that Neu1 and Neu4 should be mainly involved in the decrease of PSA in the injured brain.

### <Experiment 5: To check influence by suppressing expression of Neu1 and Neu4>

For Neu1 and Neu4, in order to examine the influence on PSA levels and migration of new neurons in the injured brain, cells around the injury were infected with a virus expressing miNeu1 or miNeu4 to suppress the gene expression. As a Comparative Example, a knockdown vector that knocks down the LacZ gene was used. The brain was infected with each virus on day 7 after injury, fixed on day 21 after injury, and observed and analyzed by immunohistochemistry.

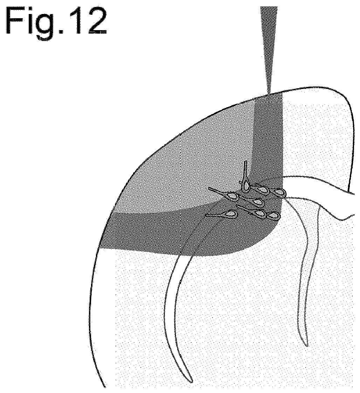

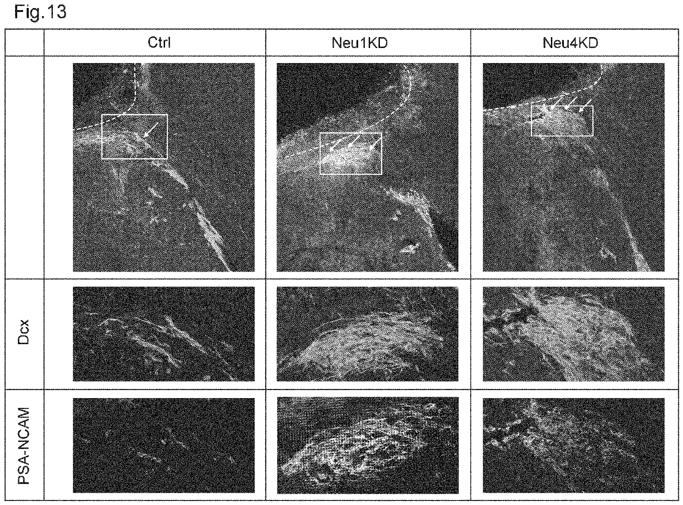

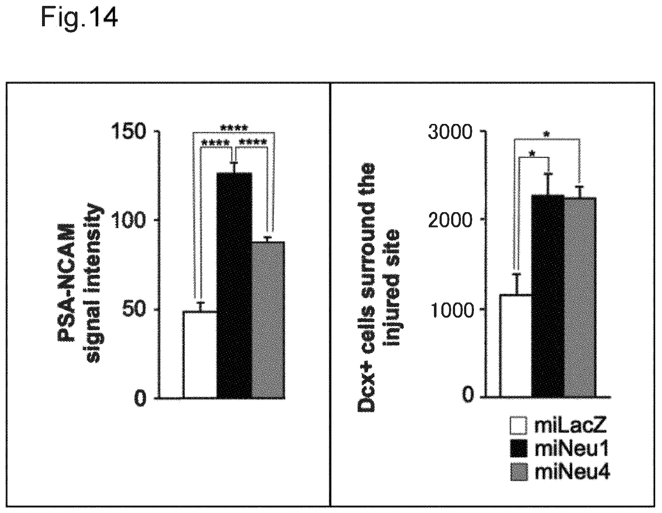

Fig. 12 is a schematic diagram showing a brain injury site of a mouse in Experiment 5. Fig. 13 is an explanatory view showing microscopic images in Experiment 5. The left side of the drawing of Fig. 13 shows a control group, the middle of the drawing shows a Neu1 knockdown group, and the right side of the drawing shows a Neu4 knockdown group. Meanwhile, in the images on the upper side of the drawing, DsRed, Dcx, and PSA-NCAM are shown, the brain injury site is indicated by a broken line, and the new neuron migrating to the injury site is indicated by an arrow. The images showing the expression of Dcx and the images showing the expression of PSA-NCAM are placed on the lower side of the drawing, and the region surrounded by the square frame in each image on the upper side of the drawing is enlarged. Fig. 14 provides graphs showing signal intensities in Experiment 5. The left side of the drawing of Fig. 14 shows the signal intensity of PSA-NCAM, and the right side of the drawing of Fig. 14 shows the signal intensity of Dcx-positive cells localized at the brain injury site.

The results shown in Fig. 13 and 14 have revealed the following. That is, in the Neu1 knockdown group and the Neu4 knockdown group, the PSA signal intensity of new neurons migrating toward the injury site significantly increased as compared with the control group. Further, in the Neu1 knockdown group and the Neu4 knockdown group, the number of new neurons migrating to the injury site significantly increased as compared with the control group. The above results have suggested that the suppression of the expression of Neu1 and the suppression of the expression of Neu4 in the injured brain can lead to the maintenance of PSA in new neurons and promote the migration of new neurons to the injury site.

### <Experiment 6: To check whether neuraminidase inhibitor administration promotes migration of new neurons>

In order to inhibit neuraminidase in a wider range of brain tissue, N-acetyl-2,3-didehydro-2-deoxyneuraminic acid (DANA) or an anti-influenza drug Zanamivir was administered as a neuraminidase inhibitor by intraperitoneal injection under conditions at 50 mg/kg for 14 consecutive days, from day 7 to day 21 after brain injury. The control group received PBS by intraperitoneal injection under the same conditions. The brain was fixed on day 21 after injury, and observed and analyzed by immunohistochemistry.

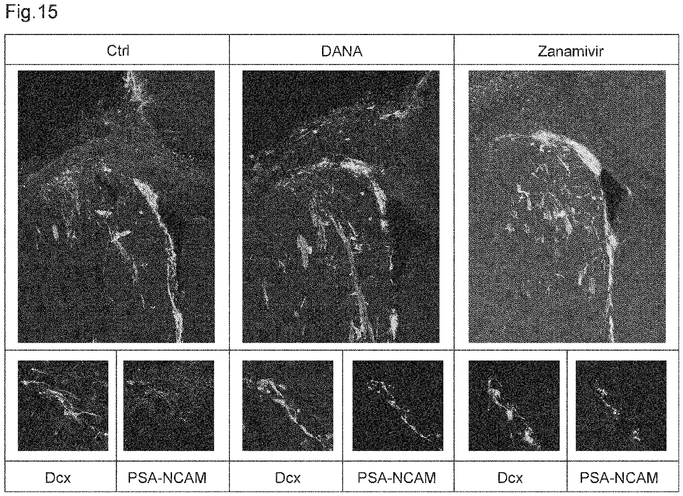

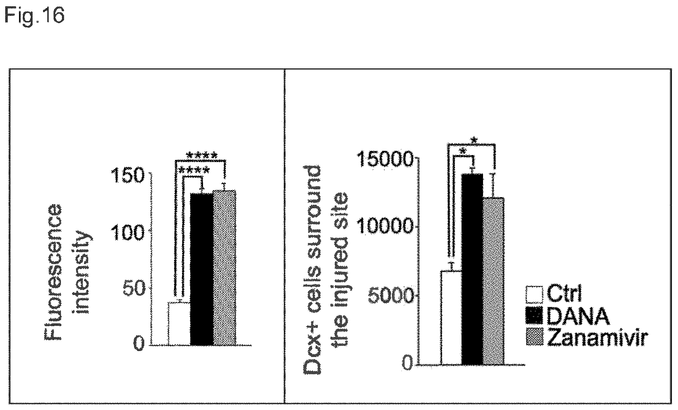

Fig. 15 is an explanatory view showing microscopic images in Experiment 6. The left side of the drawing of Fig. 15 shows a control group, the middle of the drawing shows a DANA administration group, and the right side of the drawing shows a Zanamivir administration group. In addition, the images on the upper side of the drawing show a relatively wide region ranging from the corpus callosum to the striatum and the ventricle, and the lower side of the drawing shows representative examples of an image indicating expression of Dcx and an image indicating expression of PSA-NCAM. Fig. 16 provides graphs showing signal intensities in Experiment 6. The left side of the drawing of Fig. 16 shows the signal intensity of PSA-NCAM, and the right side of the drawing of Fig. 16 shows the signal intensity of Dcx-positive cells surrounding the brain injury site.

The results shown in Fig. 15 and 16 have revealed the following. That is, in the neuraminidase inhibitor administration group, the PSA signal intensity of new neurons migrating toward the injury site significantly increased as compared with the control group. That is, in the neuraminidase inhibitor administration group, the number of new neurons migrating toward the injury site significantly increased as compared with the control group. These results suggest that administration of a neuraminidase inhibitor promotes maintenance of PSA in new neurons and migration to an injury site.

### <Experiment 7: To check whether neuraminidase inhibitor administration promotes neuronal regeneration>

To investigate whether administration of a neuraminidase inhibitor contributed to neuronal regeneration, DANA as a neuraminidase inhibitor was administered by intraperitoneal injection under conditions at 50 mg/kg for 14 consecutive days, from day 7 to day 21 after brain injury. The control group received PBS by intraperitoneal injection under the same conditions. The brain was fixed on day 35 after injury, and observed and analyzed by immunohistochemistry.

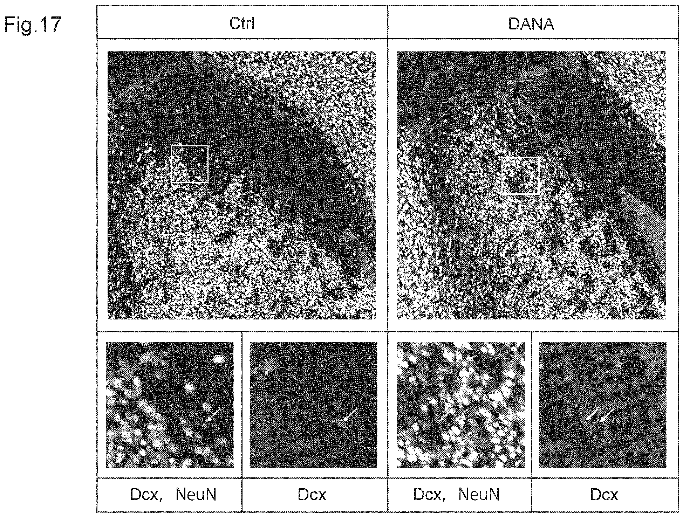

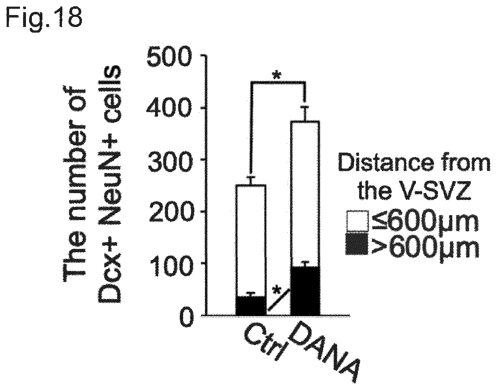

Fig. 17 is an explanatory view showing microscopic images in Experiment 7. The left side of the drawing of Fig. 17 shows a control group, and the right side of the drawing shows a DANA administration group. The images on the upper side of the drawing show Dcx and NeuN, as a mature neuron marker. The images showing the expression of Dcx and NeuN and the images showing the expression of only Dcx are placed on the lower side of the drawing, and the region surrounded by the square frame in each image on the upper side of the drawing is enlarged. In Fig. 17, Dcx-positive and NeuN-positive cells, i.e. Dcx- and NeuN-double-positive cells, are indicated by arrows. Fig. 18 is a graph showing the number of double-positive cells in Experiment 7. Fig. 18 shows the number of double-positive cells located at a distance of 600 µm or less from the ventricular-subventricular zone (V-SVZ) and the number of double-positive cells located at a distance of more than 600 µm.

The results shown in Fig. 17 and 18 have revealed the following. That is, in the neuraminidase inhibitor administration group, the number of new neurons expressing NeuN around the injury site was found to significantly increase as compared with the control group. Further, it has been found that in the neuraminidase inhibitor administration group, the number of double-positive cells distributed at a position 600 µm or more away from the subventricular zone, where new neurons are produced, significantly increased as compared with the control group. These results have suggested that the neuraminidase inhibitor administration can promote the migration of new neurons, and the new neurons reached the injury site and matured.

### <Experiment 8: To check whether neuraminidase inhibitor administration restores motor function>

It was investigated whether neuraminidase inhibitor administration contributed to restoration of a motor function lost by injury. DANA or Zanamivir as a neuraminidase inhibitor was administered by intraperitoneal injection under conditions at 50 mg/kg for 14 consecutive days, from day 7, when the migration of new neurons is assumed to start, to day 21 after brain injury. The control group received PBS by intraperitoneal injection under the same conditions. A Foot Fault Test (FFT) was performed before brain injury, 7 days after brain injury, and 35 days after brain injury. The motor function was analyzed on day 35 after brain injury, when the peak of migration of new neurons is assumed to end and the new neurons are assumed to start to mature.

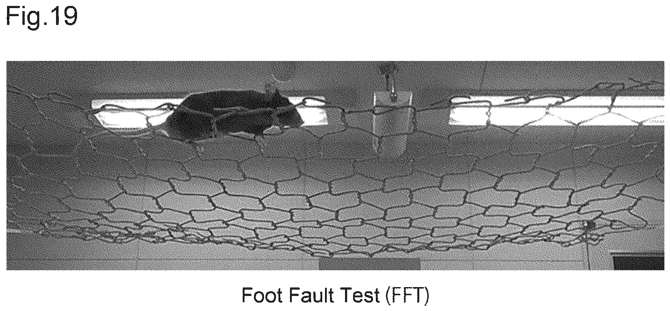

Fig. 19 is a photo illustrating how FFT looks. The FFT is one of behavior tests, and is a test for evaluating a walking function by allowing a mouse to walk on a mesh to compare how frequent the left or right limb of a mouse slips from the mesh. FFT was performed by modifying a known method (Hernandez *et al.,* 1988.). Specifically, each mouse was placed on an elevated, wire-made hexagonal grid with a 40 mm wide opening to allow free walking. In a case where the mouse slid or the limb dropped into the gap of the grid and fell down, a misstep was recorded as a foot fault. The number of foot faults in each limb was counted for 10 minutes, and the ratio of the number of faults in the contralateral (left) forelimb and hindlimb to the total number of faults for four limbs was determined as a percentage.

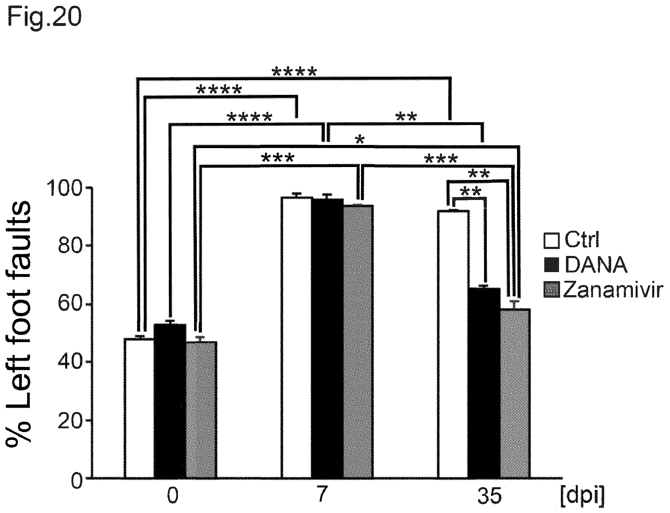

Fig. 20 is a graph showing the results of Experiment 8. The vertical axis in Fig. 20 indicates the rate at which the left forelimb and hindlimb slip off the mesh. The results shown in Fig. 20 have revealed the following. That is, in both the neuraminidase inhibitor administration group and the control group, the rate of the left forelimb and hindlimb slipping off from the mesh was about 50% before the injury, and after 7 days from the injury, the rate increased in both groups from before the injury. By contrast, 35 days after the injury, the left slipping-off rate was significantly lower in the neuraminidase inhibitor administration group than in the control group. The above results have suggested that restoration of the motor function lost due to cerebral infarction or the like is promoted by administering a neuraminidase inhibitor during a period in which migration of new neurons is active.

### <Experiment 9: To quantify sialic acid in injured brain>

An additional experiment to Experiment 6 was conducted by quantitatively analyzing, using HPLC (high performance liquid chromatography), the concentration of sialic acid (Neu5Ac) in Area 1 and Area 2 at different distances from the injury site as well as in the rostral migratory stream (RMS) of the normal brain. The n number of each of the DANA administration group and the Zanamivir administration group was set to 4, the n number of the control group having received PBS instead of the neuraminidase inhibitor was set to 3, and the Tukey test was used for multiple comparison. Note that the error bars indicate the mean ± standard error of the mean.

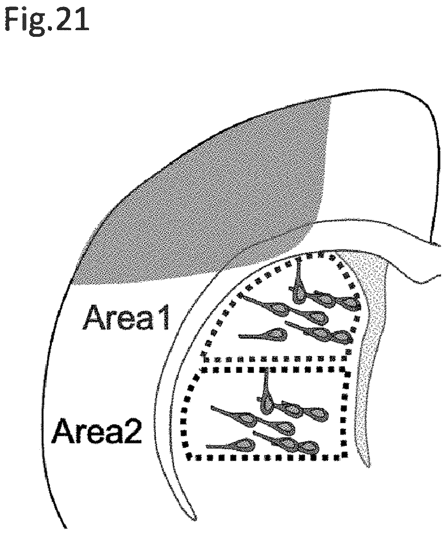

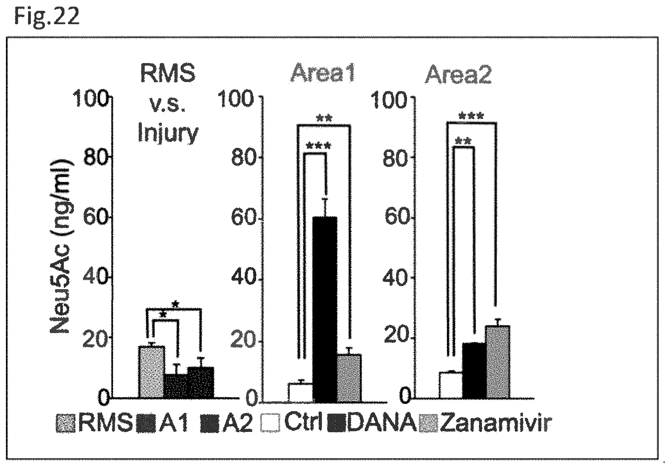

Fig. 21 is a schematic diagram illustrating brain areas used for quantification of sialic acid in Experiment 9. Fig. 22 provides graphs showing the results of sialic acid quantification in Experiment 9. In Fig. 22, the left side of the drawing shows the comparison between the RMS and the injured brain areas, the middle of the drawing shows the comparison between the respective groups in Area 1, and the right side of the drawing shows the comparison between the respective groups in Area 2. The results shown in Fig. 22 have revealed the following. That is, sialic acid concentrations in the injured brain areas (Area 1 and Area 2) were significantly lower than the sialic acid concentration in normal RMS. In addition, the administration of each neuraminidase inhibitor caused an increase in sialic acid in both Area 1 and Area 2. These results have revealed that PSA was decreased by injury, but sialic acid was increased by administration of a neuraminidase inhibitor in any of injury Areas.

### <Experiment 10: Co-culture of V-SVZ-derived new neurons with BV2 cells>

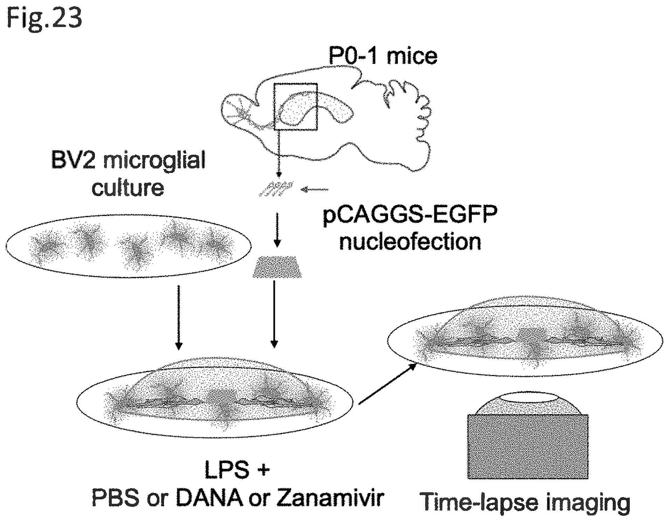

Fig. 23 is a scheme illustrating an outline of Experiment 10. In order to investigate whether neuraminidase inhibitor administration promoted neuronal migration, new neurons and the microglial cell line BV2 were co-cultured as an *in vitro* system that mimics the injured brain. The microglial cell line BV2 has been reported to release neuraminidase upon LPS stimulation (J. Biol. Chem. 290, 13202-14 (2015)). Thus, co-culture was performed to examine the PSA signal intensity after the addition of LPS, and also examine the migration speed of new neurons by time-lapse imaging. In the following description, "LPS-" indicates an LPS-free control, and "LPS+" indicates that LPS was added. In the analysis of the PSA signal intensity, the n number of control (LPS-) was set to 6, and the n number of each of PBS (LPS+), DANA (LPS+), and Zanamivir (LPS+) was set to 10. In the time-lapse imaging, the n number of control (LPS-) was set to 8, and the n number of each of PBS (LPS+), DANA (LPS+), and Zanamivir (LPS+) was set to 30. In all cases, Mann-Whitney U test was used for multiple comparison. Note that the error bars indicate the mean ± standard error of the mean.

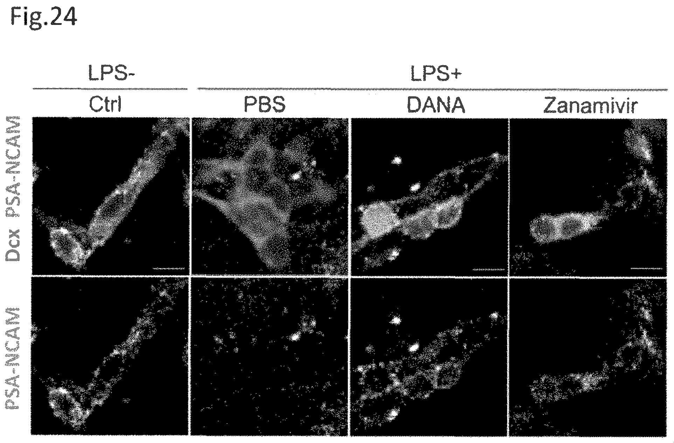

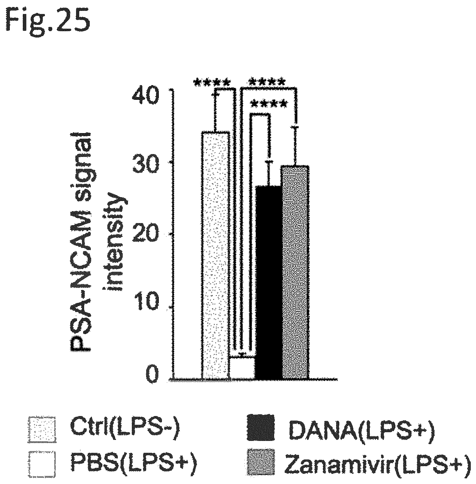

Fig. 24 is an explanatory view showing representative microscopic images in Experiment 10. The upper side of the drawing of Fig. 24 is each image showing the expression of Dcx and PSA-NCAM, and the lower side of the drawing is each image showing only the expression of PSA-NCAM. Fig. 25 is a graph showing PSA signal intensities in Experiment 10. The results shown in Fig. 24 and 25 have revealed the following. That is, as can be seen from the comparison between the control (LPS-) and PBS (LPS+), the PSA signal was decreased by the LPS stimulation, but the PSA signal intensity was maintained by adding DANA or Zanamivir as a neuraminidase inhibitor.

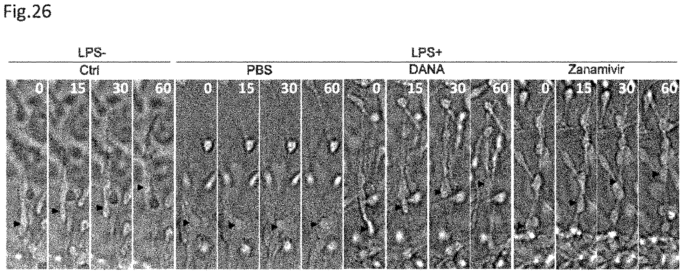

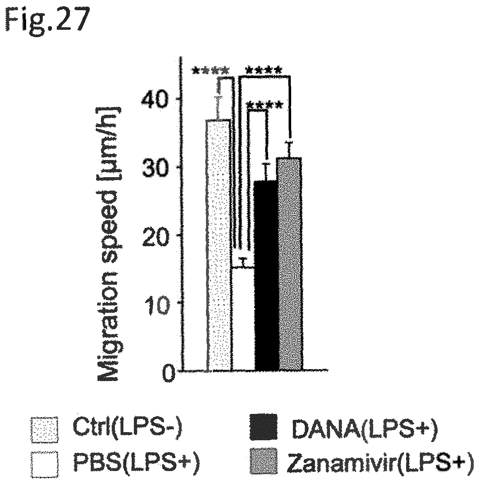

Fig. 26 is an explanatory view showing a result of time-lapse imaging in Experiment 10. In Fig. 26, the number attached to the upper side of each time-lapse image indicates at which minutes after the first imaging frame the image was captured. In addition, in Fig. 26, the position of a new neuron is indicated by an arrowhead. Fig. 27 is a graph showing the migration speed of neurons in Experiment 10. The results shown in Fig. 26 and 27 have revealed the following. That is, it has been revealed that the migration speed of neurons was reduced by LPS stimulation, but the migration speed of neurons was recovered by adding a neuraminidase inhibitor. The above results have suggested that the neuraminidase inhibitor administration can promote the migration of new neurons while PSA in new neurons is maintained.

### <Experiment 11: To check whether neuraminidase inhibitor administration promotes regeneration of neurons>

As an additional experiment of Experiment 7, an experiment using DANA or Zanamivir as a neuraminidase inhibitor was performed. The neuraminidase inhibitor was administered by intraperitoneal injection under conditions at 50 mg/kg for 14 days from day 7 after the brain injury. The brain was fixed on day 35 after injury, and observed and analyzed by immunohistochemistry.

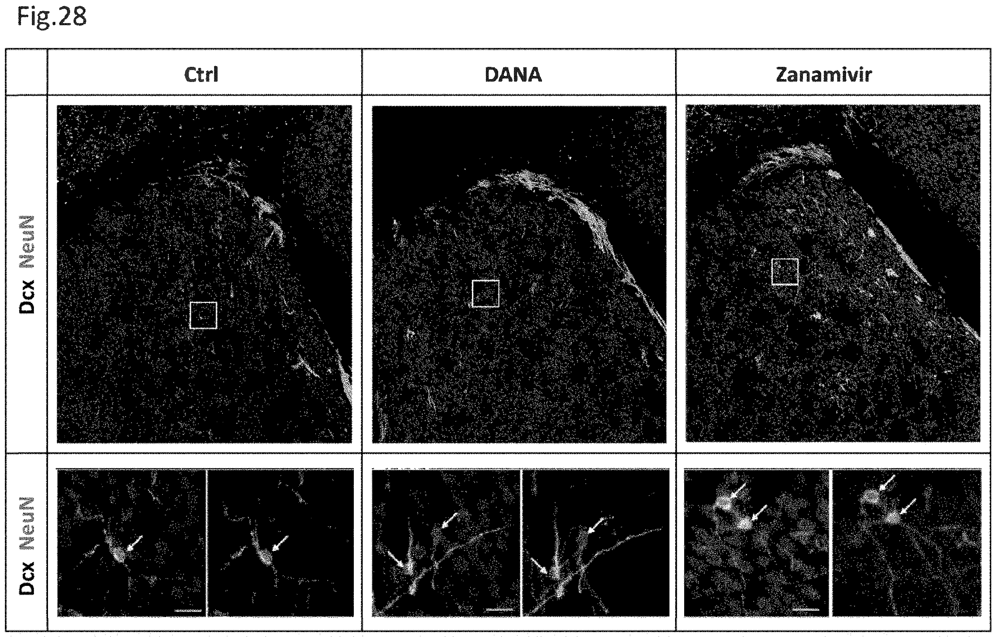

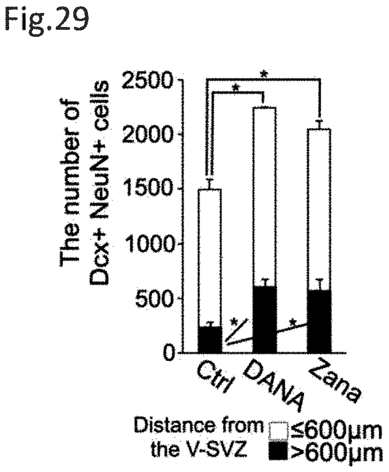

Fig. 28 is an explanatory view showing representative microscopic images in Experiment 11. The left side of the drawing of Fig. 28 shows a control group, the middle of the drawing shows a DANA administration group, and the right side of the drawing shows a Zanamivir administration group. The images on the upper side of the drawing show Dcx and NeuN. The images showing the expression of Dcx and NeuN and the images showing the expression of only Dcx are placed on the lower side of the drawing, and the region surrounded by the square frame in each image on the upper side of the drawing is enlarged. In Fig. 28, Dcx- and NeuN-double-positive cells are indicated by arrows. The Dex- and NeuN-double-positive cells indicate a mature form with long branched protrusions. Fig. 29 is a graph showing the number of double-positive cells in Experiment 11. Fig. 29 shows the number of double-positive cells located at a distance of 600 µm or less from the V-SVZ and the number of double-positive cells located at a distance of more than 600 µm.

The results shown in Fig. 28 and 29 have revealed the following. That is, like Experiment 7, in the neuraminidase inhibitor administration group, the number of new neurons expressing NeuN around the injury site significantly increased as compared with the control group. In addition, like Experiment 7, in the neuraminidase inhibitor administration group, the number of double-positive cells distributed at a position 600 µm or more away from the V-SVZ significantly increased as compared with the control group. The results of Experiment 11 have demonstrated that the neuraminidase inhibitor administration can promote the migration of new neurons in an injured brain, and the new neurons can reach the injury site and mature.

### <Experiment 12: To check whether restoration of motor function is caused by neuronal regeneration>

According to Experiment 8 described above, restoration of a motor function was found to be caused by administration of a neuraminidase inhibitor. Thus, next, it was examined whether the functional recovery observed in inhibitor-administered animals was due to regeneration of V-SVZ-derived neurons. For this purpose, a system for specifically removing new neurons (Nestin-CreERT2; NSE-DTA) was used. Tamoxifen was injected 8 times from 20 days before injury into Nestin-CreERT2; NSE-DTA mice to express diphtheria toxin in a neuron-specific manner, thereby inducing cell death. Thereafter, the brain was injured. Zanamivir was administered by intraperitoneal injection under conditions at 50 mg/kg for 14 days from day 7 after injury. Fourteen days after the end of Zanamivir administration, a behavior test (FFT) was performed, and Dcx- and NeuN-double-positive cells were analyzed. Note that as a control, NSE-DTA mice were used. The n number of each group was set to 3, and t-test was used. Note that the error bars indicate the mean ± standard error of the mean.

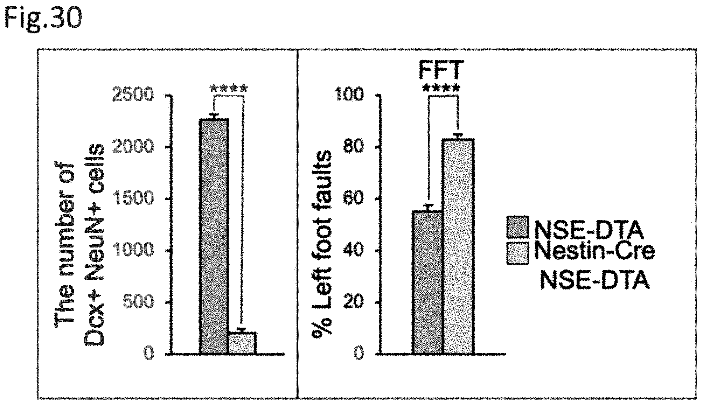

Fig. 30 provides graphs showing the results of Experiment 12. In Fig. 30, the left side of the drawing shows the number of double-positive cells, and the right side of the drawing shows the results of FFT. As shown in Fig. 30, the Nestin-CreERT2;NSE-DTA group, unlike the NSE-DTA group, exhibited a decrease in the number of the Dcx- and NeuN-double-positive cells even when the neuraminidase inhibitor was administered. Further, the Nestin-CreERT2;NSE-DTA group, unlike the NSE-DTA group, exhibited no improvement of the left-side slipping-off rate in the FFT even by administration of Zanamivir. These results indicate that V-SVZ-derived neurons regenerated in the injured brains of mice having received a neuraminidase inhibitor contribute to function restoration.

### <Experiment 13: To check effect of neuraminidase inhibitor administration in primates>

As an additional experiment of Experiment 6, it was checked whether the effect of neuraminidase inhibitor administration in an injured brain on neuronal regeneration was also observed in animal species closer to humans. Primate common marmosets were given brain injury and Zanamivir was administered by intraperitoneal injection under conditions at 50 mg/kg for 21 days from day 7 after injury. Here, 28 days after the injury, the brain was fixed and analyzed. In immunostaining, the n number of each of the normal RMS group, the Zanamivir administration group, and the control group was set to 15, and the Mann-Whitney U test was used for multiple comparison. The migration distance of Dcx-positive cells from the injury site in the brain was analyzed using the Wilcoxon signed rank test for multiple comparison while the n number of the control group was set to 148 and the n number of the Zanamivir administration group was set to 140. Note that the error bars indicate the mean ± standard error of the mean.

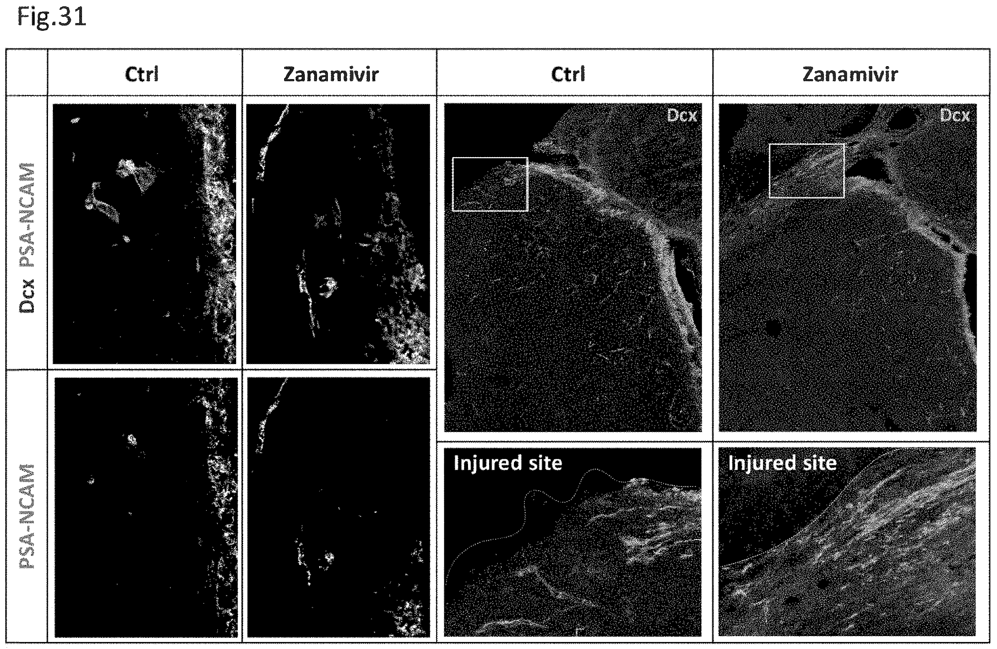

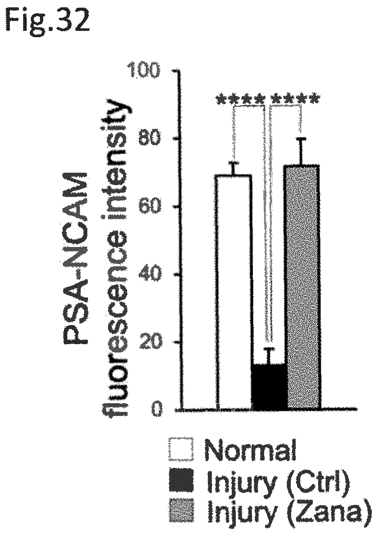

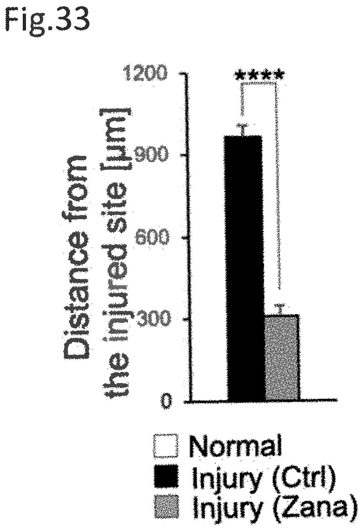

Fig. 31 is an explanatory view showing representative microscopic images in Experiment 13. In Fig. 31, the expression of Dcx and the expression of PSA-NCAM are shown on the upper left side of the drawing for each of the control group and the Zanamivir administration group. The lower left side of the drawing shows the expression of PSA-NCAM, and the upper right side of the drawing shows each representative image of a common marmoset brain section stained with Dcx. On the lower right side of the drawing, a region surrounded by a square frame in each image on the upper right side of the drawing is enlarged. Fig. 32 is a graph showing PSA-NCAM signal intensity in Experiment 13. Fig. 32 shows the PSA-NCAM fluorescence intensities of Dex-positive cells in normal RMS and in the injured brain control and Zanamivir administration groups. Fig. 33 is a graph showing the migration distance of Dcx-positive cells from the injury site in the brain.

The results shown in Figs. 31 to 33 have revealed the following. That is, the control (Zanamivir non-administration group) had a lower PSA signal intensity of new neurons localized in the striatum on the injury side than the PSA signal intensity of new neurons localized in the RMS. However, it has been revealed that the administration of Zanamivir can help maintain the PSA signal intensity of new neurons localized in the striatum on the injury side. Further, it has been demonstrated that by administration of Zanamivir, new neurons are localized closer to the injury site. These results have suggested that even in primates, like mice, PSA is maintained and neuronal migration to an injury site is promoted by administration of a neuraminidase inhibitor in new neurons migrating to the brain injury site.

### [Industrial Applicability]

The present invention makes it possible to promote migration of neurons, so that the number of neurons migrating to an injury site can be increased. The present invention should exert an effect of improving the brain function by differentiated and mature nerve cells that reach the injury site. Therefore, the present invention can be used to promote, for example, by administration to a patient with a brain disease such as cerebral infarction, migration of nerve cells produced from endogenous neural stem cells. In addition, it can be used to promote migration of transplanted nerve cells, for example, by administration to a patient who has undergone cell transplantation surgery. Further, the present invention may be used in combination with, for example, a technology such as biomaterial to promote migration and regeneration of nerve cells. Furthermore, it can be administered as a neuronal migration promoter to experimental animals such as brain disease model animals to be used for research.

The present invention is not limited to the above-described embodiments, and can be realized by various configurations without departing from the spirit of the present invention. For example, the technical features in the embodiments and Examples corresponding to the technical features in the respective embodiments described in the Summary of Invention section can be appropriately replaced or combined in order to solve part or all of the above-described problems or achieve part or all of the above-described effects. Besides, when the technical features are not herein described as essential, they can be deleted if appropriate.

## Claims

1. A pharmaceutical composition for treating a brain disease, comprising a neuraminidase inhibitor.

2. The pharmaceutical composition according to claim 1, wherein the neuraminidase inhibitor inhibits at least one of Neu1 or Neu4.

3. The pharmaceutical composition according to claim 1 or 2, wherein administration starts before a lapse of 10 days from an onset of the brain disease and is performed daily.

4. The pharmaceutical composition according to claim 3, wherein the pharmaceutical composition is administered for 10 days or longer.

5. The pharmaceutical composition according to claim 1 or 2, wherein the pharmaceutical composition is administered in a form of an injection.

6. A neuronal migration promoter comprising a neuraminidase inhibitor.

7. The neuronal migration promoter according to claim 6, wherein the neuraminidase inhibitor inhibits at least one of Neu1 or Neu4.

8. A method of treating a brain disease, comprising a step of administering a neuraminidase inhibitor to a brain disease patient.

9. A method of promoting migration of neurons, comprising a step of bringing a neuraminidase inhibitor into contact with the neurons.

10. Use of a neuraminidase inhibitor for treatment of a brain disease.
